# FASCICULE DE BREVET EUROPEEN

(11) **EP 1 273 362 B1**
(45) Date de publication et mention de la délivrance du brevet: **14.09.2005**
(21) Numéro de dépôt: 02364032.9
(22) Date de dépôt: 27.06.2002
(51) Int. Cl.: B09B 3/00, C02F 11/00, A61L 2/04

(54) **Procédé et filière de traitement des farines et/ou graisses animales**
Verfahren und Reaktor zur Behandlung von Tiermehlen und/oder -fetten
Method and reactor for treating animal meals and/or fats

(30) Priorité: 06.07.2001 FR 0109049
(43) Date de publication de la demande: 08.01.2003
(73) Titulaire: OTV SA, 94417 Saint-Maurice Cedex (FR)
(72) Inventeur: Cretenot, Didier, 60300 Senlis (FR); Sibony, Jacques, 75014 Paris (FR)
(74) Mandataire: Larcher, Dominique

(56) Documents cités:
- EP-A- 0 908 416
- EP-A- 1 021 958
- WO-A-98/17950
- DE-A- 3 402 215
- DE-A- 19 623 163
- DE-A- 19 628 521
- DE-A- 19 713 395
- FR-A- 2 784 387
- DATABASE WPI Section Ch, Week 199401 Derwent Publications Ltd., London, GB; Class E19, AN 1994-002044 [01] XP002192174 & HU 64 104 A (PROCALOR KOERNYEZETVEDELMI ES ENERGETIKA), 29 novembre 1993 (1993-11-29)

## Description

L'invention concerne le domaine du traitement des farines et/ou graisses animales.

Plus précisément, l'invention concerne un procédé et une filière de traitement thermique des farines et/ou graisses animales.

Les farines et/ou graisses animales sont issues de sous-produits provenant des animaux d'élevage tués dans les abattoirs. Les farines sont obtenues par broyage et séchage de ces sous-produits.

Pendant de nombreuses années, une grande partie de ces farines et/ou graisses animales, riches en protéines, ont été utilisées comme additifs alimentaires en alimentation animale.

L'apparition chez l'être humain d'une nouvelle forme de la maladie de Kreutzfeld-Jacob fortement suspectée d'avoir pour cause un élément infectieux se présentant sous la forme d'une protéine altérée (prion) transmise par la consommation de bovins ayant eu une telle alimentation, a mis un terme à ce moyens de valorisation des farines et/ou graisses animales. Les pouvoirs publics ont concomitamment ordonné la destruction du pouvoir pathogène des farines et/ou graisses animales.

Dans ce but, il a été proposé d'incinérer ces farines et/ou graisses animales, notamment dans les fours des cimenteries. Toutefois, cette filière ne peut absorber la totalité de la production actuelle de farines et/ou graisses animales.

La demande de brevet WO 98/17950 décrit un procédé de mélange de farines avec des déchets urbains.

Il a aussi été proposé d'inerter ces farines et/ou graisses animales en les incluant dans certains matériaux tels que les bétons phénoliques. Toutefois, de telles voies de valorisation restent marginales.

Il existe donc actuellement de gros excédents de farines et/ou graisses animales dont le stockage dans l'attente d'un traitement, au mieux en entrepôts fermés, au pire en décharge à ciel ouvert, induit de nombreux risques, notamment sanitaires.

De nouvelles solutions à la destruction de ces farines sont donc recherchées de façon urgente.

Plus particulièrement, les abattoirs ou installations d'équarrissage qui intègrent des équipements pour incinérer les farines qu'ils produisent, vont être amenés soit à augmenter leur capacité d'incinération, soit à faire appel à des tiers pour procéder à l'élimination des farines qu'ils ne peuvent incinérer eux-mêmes.

Le document DE 19628521 A concerne un procédé de traitement de cadavres homogénéisés d'animaux dans lequel ces cadavres sont stérilisés.

L'invention a pour objectif de fournir une solution nouvelle d'élimination des farines et/ou graisses animales dans le contexte de leur retrait de la filière alimentaire.

Un autre objectif de l'invention est de proposer un procédé qui s'intègre dans une approche globale du traitement des déchets et rejets produits par les installations d'abattage et/ou d'équarrissage.

Le but est aussi d'améliorer la biodégradabilité du mélange qui par la suite est traité par une digestion aérobie ou anaérobie.

Ces objectifs ainsi que d'autres qui apparaîtront par la suite, sont atteints grâce à l'invention qui concerne un procédé de traitement des farines et/ou graisses animales, comprenant les étapes consistant à :
- mélanger lesdites farines et/ou graisses animales avec des boues résiduaires provenant de stations d'épuration des eaux usées (urbaines ou industrielles) ;
- faire subir un traitement thermique d'hygiénisation audit mélange à une température supérieure à 135°C.

Une telle température supérieure à 135°C est suffisante pour hygiéniser ce mélange et notamment selon les règles de l'OMS pour détruire les prions pouvant être contenus dans celui-ci.

On obtient ainsi un procédé de traitement des farines et/ou graisses simultanément au traitement thermique opéré sur des boues résiduaires de stations d'épuration permettant d'obtenir un ou des produits hygiénisés c'est-à-dire ne présentant aucun pouvoir de nuisance pour la santé humaine ou l'environnement.

Les farines et les graisses sont, selon ce procédé, totalement hygiénisées en subissant un traitement thermique qui détruit les germes et/ou prions qu'elles peuvent contenir.

Le procédé est particulièrement adapté aux installations d'abattage ou d'équarrissage qui incluent souvent des moyens de traitement de leurs effluents, et notamment une station d'épuration.

Selon une solution préférée, ledit mélange comprend en poids entre environ 10 à 40 % de farines et/ou graisses animales.

De telles proportions permettent d'éliminer des quantités importantes de farines et/ou graisses animales. Dans la pratique, la quantité de farines et/ou graisses animales introduites dans le mélange sera fonction de la teneur en matières sèches des boues résiduaires. Ainsi, plus la teneur en matières sèches des boues résiduaires sera faible, plus la quantité de farines et/ou graisses animales introduites dans le mélange pourra être élevée.

Selon une solution avantageuse, ladite étape de traitement thermique comprend au moins une étape d'hydrolyse thermique dudit mélange par injection de vapeur d'eau dans celui-ci. Cette hydrolyse s'effectue de préférence à 165°C durant 30 minutes à 1 heure.

D'autres techniques de traitement thermique sont bien entendu envisageables dans d'autres modes de réalisation sans sortir du cadre de l'invention.

Avantageusement, ladite étape d'hydrolyse thermique est suivie par au moins une étape de digestion des boues hydrolysées conduisant à l'obtention d'un biogaz et de boues digérées.

L'obtention d'un tel biogaz peut être mise à profit comme cela va être décrit par la suite, dans la mesure où le procédé s'intègre dans une installation visant à traiter également les effluents aqueux provenant d'un abattoir et/ou d'une installation d'équarrissage.

Egalement selon une solution avantageuse, ladite étape de traitement thermique comprend une oxydation par voie humide.

On notera que la méthode d'oxydation par voie humide a été abondamment décrite dans l'art antérieur. Cette technique vise à réaliser une oxydation de la matière organique peu biodégradable contenue dans des effluents aqueux, par contact de ces effluents avec un gaz oxydant à température élevée, tout en maintenant les effluents à l'état liquide.

Selon une première approche, ladite étape d'oxydation par voie humide est réalisée sur lesdites boues digérées obtenues à l'issue des étapes d'hydrolyse et de digestion citées ci-dessus.

Selon une autre approche, ladite étape d'oxydation par voie humide est réalisée directement sur ledit mélange de boues de stations d'épuration des eaux usées et de farines et/ou graisses animales.

Dans l'un ou l'autre cas, ladite étape d'oxydation par voie humide est réalisée à une température comprise entre environ 150 °C et environ 300 °C et à une pression comprise entre environ 15 bar et environ 160 bar.

L'invention concerne également une filière de traitement des effluents provenant d'abattoirs intégrant une station d'épuration constitués d'une part par des boues provenant de ladite station d'épuration et d'autre part par des effluents aqueux, la filière de traitement comprenant, selon l'invention, une installation de traitement desdites boues conjointement à des farines et/ou graisses animales grâce au procédé tel que décrit précédemment, ladite installation comprenant des moyens de traitement thermique du mélange boues/farines incluant au moins un digesteur conduisant à la formation de biogaz et des moyens d'acheminement dudit biogaz vers lesdits effluents aqueux pour hygiéniser ceux-ci.

D'autres caractéristiques et avantages de l'invention apparaîtront plus clairement à la lecture de la description suivante d'un mode de réalisation de invention donné à titre d'exemple illustratif et non limitatif, en référence à la figure unique qui illustre de façon schématique le procédé de traitement de farines et/ou graisses animales selon l'invention.

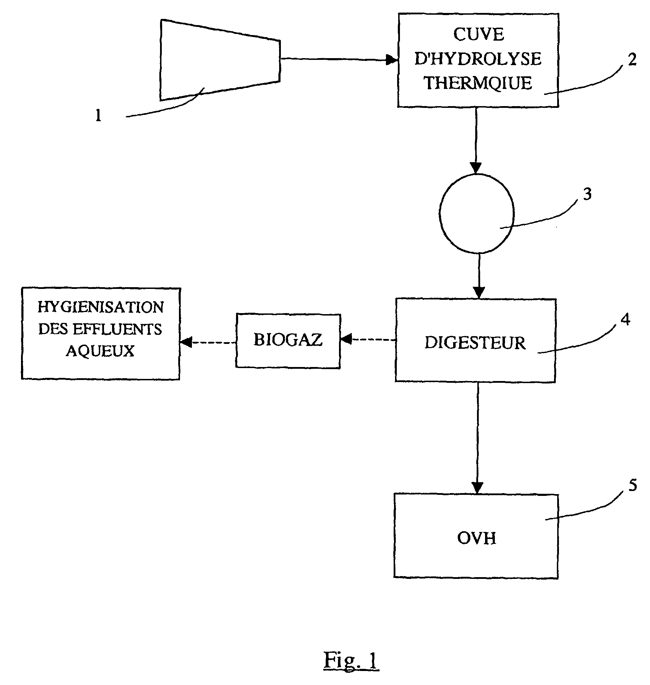

En référence à la figure 1, le procédé selon l'invention a été testé grâce à une installation comprenant les éléments fonctionnels représentés de façon schématique sur cette figure.

A l'aide du mélangeur 1, on procède à un mélange comprenant en poids sec environ 76,5 % de boues provenant d'une station d'épuration et environ 24 % en farines et/ou graisses animales.

Au cours de tests effectués, les boues primaires et secondaires de station d'épuration sont déshydratées et produites à raison de 8937 kg de matières sèches (MS) par jour. Elles présentent les caractéristiques suivantes :
- matières sèches (MS) : 14 %
- matières volatiles (MV) : 65 %

Les farines et/ou graisses animales représentent quant à elles 2750 kg de matières sèches (MS) par jour et présentent les caractéristiques suivantes :
- matières sèches (MS) : 93 %
- matières volatiles (MV) : 70 %

Les boues déshydratées et les farines et/ou graisses animales sont mélangées. On obtient donc une production journalière de 11687 kg de boues déshydratées présentant les caractéristiques suivantes :
- concentration en MS : 180 g/l
- concentration en matières volatiles en suspension (MVS) : 66 %
- Azote total (NTK) : 5,47 g/l
- Azote ammoniacal (N-NH₄): 0,15 g/l
- Phosphore total (PT) : 5,33 g/l
- Demande Chimique en Oxygène (DCO) particulaire : 211 g/l

Ces boues déshydratées sont acheminées dans un réacteur 2 où elles subissent une hydrolyse thermique pendant 30 à 60 minutes à une température de 165 °C, à l'aide d'une injection dans le réacteur 2 de vapeur d'eau à 195 °C sous une pression d'au moins 17 bar selon un débit moyen de 0,52 t/h.

Les boues déshydratées hydrolysées sont ensuite refroidies à l'aide d'un échangeur thermique 3, jusqu'à une température de 40 °C.

En sortie d'hydrolyse, les boues représentent une quantité de 7831 kg MS/jour pour un débit de 3,32 m³/h. Les boues hydrolysées avant leur introduction dans le digesteur 4, présentent les caractéristiques suivantes :
- concentration MS : 98,29 g/l
- MVS particulaire : 49,25 %
- concentration MS particulaire : 48,41 g/l
- concentration MV soluble : 48,41 g/l
- NTK : 3,74 g/l
- N-NH₄: 0,84 g/l
- PT: 4,35 g/l
- DCO particulaire : 91,11 g/l
- DCO soluble : 80,80 g/l

La digestion des boues hydrolysées en milieu anaérobie entraîne l'obtention d'un biogaz comprenant en volume 65 % de méthane, avec un débit de 7706 Nm³/par jour.

Comme indiqué sur la figure 1, ce biogaz est utilisé pour chauffer les effluents aqueux provenant de l'abattoir intégrant une installation de traitement des farines et des boues selon l'invention. Ces effluents aqueux sont ainsi hygiénisés.

Le biogaz est aussi utilisé pour produire la vapeur nécessaire à l'hydrolyse thermique.

En sortie du digesteur, on peut mélanger des boues dites tertiaires en provenance d'une déphosphatation physico-chimique située après le traitement biologique, avec les boues digérées.

Les boues dites tertiaires représentent une production journalière de 3011 kg MS pour un débit moyen de 1,93 m³/h, et présentent les caractéristiques suivantes :
- concentration MS : 65 g/l
- MVS particulaire : 37 %
- concentration MV particulaire : 24,05 g/l
- NTK : 4,00 g/l
- N- NH₄ : 0,1 g/l
- PT : 8,55 g/l

Les boues digérées représentent une production journalière de 5894 kg MS pour un débit moyen de 3,13 m³/h, et présentent les caractéristiques suivantes :
- concentration MS : 78,41 g/l
- MVS particulaire : 34,64 %
- concentration MV particulaire : 27,16 g/l
- concentration MV soluble : 15,62 g/l
- NTK : 3,96 g/l
- N- NH₄ : 2,31 g/l
- PT : 4,92 g/l
- DCO particulaire : 52,02 g/l
- DCO soluble : 19,83 g/l

Le mélange des boues de la station d'épuration avec les boues digérées représente ainsi une production journalière de 8905 kg MS pour un débit de 8 m³/h et présentent les caractéristiques suivantes :
- concentration MS : 73,29 g/l
- MVS particulaire : 35,44 %
- concentration MV particulaire : 25,98 g/l
- concentration MV soluble : 9,66 g/l
- MV total/MS : 48,62 %
- NTK : 3,98 g/l
- N- NH₄: 1,47 g/l
- PT : 6,30 g/l
- DCO total : 66,66 g/l

Le mélange des boues est ensuite acheminé vers l'installation d'oxydation par voie humide 5 dans laquelle elles sont traitées selon les conditions opératoires suivantes :
- température : 235 °C
- pression : 42 bars

L'oxygène est introduit dans l'installation OVH selon un débit de 373 Nm³/h d'oxygène pur..

Après l'oxydation par voie humide, on obtient d'une part une matière solide inerte et totalement hygiénisée et, d'autre part, un effluent aqueux aisément biodégradable à faible teneur en azote.

La matière solide est produite à raison de 7167 kg MS/jour, et présente une concentration en MS de 50 % pour moins de 5 % de matière organique particulaire. Cette matière solide se présente sous la forme d'un sable inerte totalement hygiénisé et ne présentant aucun danger pour la santé humaine ou pour l'environnement.

Les effluents aqueux en sortie d'OVH présentent un débit de 9,87 m³/h et les caractéristiques suivantes :
- température : 45 °C
- concentration MS : 0,50 g/l
- MVS particulaire : 3,65 %
- DCO total : 3,58 g/l
- NTK : 1,53 g/l
- N- NH₄ : 1,21 g/l

D'autres modes de réalisation utilisant le principe du procédé de traitement de farines et/ou graisses animales conjointement à des boues résiduaires de station d'épuration sont bien entendu envisageables sans sortir du cadre de l'invention, selon les revendications indépendantes 1 et 9.

## Revendications

1. Procédé de traitement des farines et/ou graisses animales, comprenant les étapes consistant à :
- mélanger lesdites farines et/ou graisses animales avec des boues résiduaires provenant de stations d'épuration des eaux usées ;
- faire subir un traitement thermique d'hygiénisation audit mélange à une température supérieure à 135°C.

2. Procédé de traitement de farines et/ou graisses animales selon la revendication 1 **caractérisé en ce que** ledit mélange comprend en poids sec entre environ 10 % et 40 % de farines et/ou graisses animales

3. Procédé de traitement de farines et/ou graisses animales selon les revendication 1 ou 2, **caractérisé en ce que** ladite étape de traitement thermique comprend au moins une étape d'hydrolyse thermique dudit mélange par injection de vapeur d'eau dans celui-ci.

4. Procédé de traitement de farines et/ou graisses animales selon la revendication 3, **caractérisé en ce que** ladite étape d'hydrolyse thermique est suivie par au moins une étape de digestion des boues hydrolysées conduisant à l'obtention d'un biogaz et de boues digérées.

5. Procédé de traitement de farines et/ou graisses animales selon l'une des revendications 1 à 4, **caractérisé en ce que** ladite étape de traitement thermique comprend une oxydation par voie humide.

6. Procédé de traitement de farines et/ou graisses animales selon les revendications 4 et 5, **caractérisé en ce que** ladite étape d'oxydation par voie humide est réalisée sur lesdites boues digérées.

7. Procédé de traitement de farines et/ou graisses animales selon la revendication 5, **caractérisé en ce que** ladite étape d'oxydation par voie humide est réalisée sur ledit mélange.

8. Procédé de traitement de farines et/ou graisses animales selon l'une quelconque des revendications 5 à 7, **caractérisé en ce que** ladite étape d'oxydation par voie humide est réalisée à une température comprise entre 150 °C et environ 300 °C et à une pression comprise entre 15 bar et 160 bar.

9. Filière de traitement des effluents provenant d'abattoirs intégrant une station d'épuration (1), lesdits effluents étant constitués d'une part par des boues provenant de ladite station d'épuration et d'autre part par des effluents aqueux ladite filière comprenant une installation de traitement desdites boues conjointement à des farines et/ou graisses animales grâce au procédé selon les revendications 4 à 8, ladite installation comprenant des moyens de traitement thermique (2) du mélange boues/farines incluant au moins un digesteur (4) conduisant à la formation de biogaz et des moyens d'acheminement dudit biogaz vers lesdits effluents aqueux pour hygiéniser ceux-ci.

## Patentansprüche

1. Verfahren zur Behandlung von Tiermehlen und/oder tierischen Fetten, umfassend die Schritte bestehend aus:
- Mischen der Tiermehle und/oder tierischen Fette mit Klärschlämmen, die Klärstationen von Schmutzwässem entstammen;
- Unterziehen der Mischung einer thermischen Säuberungsbehandlung bei einer Temperatur größer als 135°C.

2. Behandlungsverfahren nach Anspruch 1, **dadurch gekennzeichnet, dass** die Mischung im Trockengewicht zwischen etwa 10% und 40% an Tiermehlen und/oder tierischen Fetten umfasst.

3. Verfahren nach einem der Ansprüche 1 oder 2, **dadurch gekennzeichnet, dass** der thermische Behandlungsschritt zumindest einen thermischen Hydrolyseschritt der Mischung durch Injektion von Wasserdampf in die Mischung umfasst.

4. Behandlungsverfahren nach Anspruch 3, **dadurch gekennzeichnet, dass** der thermische Hydrolyseschritt gefolgt wird von zumindest einem Verdauungsschritt der hydrolysierten Schlämme, durchgeführt zum Erhalten eines Biogases und verdauter Schlämme.

5. Behandlungsverfahren nach einem der Ansprüche 1 bis 4, **dadurch gekennzeichnet, dass** der thermische Behandlungsschritt eine Oxidation auf feuchtem Wege umfasst.

6. Behandlungsverfahren nach einem der Ansprüche 4 und 5, **dadurch gekennzeichnet, dass** der Schritt der Oxidation auf feuchtem Wege angewandt wird auf die verdauten Schlämme.

7. Behandlungsverfahren nach Anspruch 5, **dadurch gekennzeichnet, dass** der Schritt der Oxidation auf feuchtem Wege angewandt wird auf die Mischung.

8. Verfahren nach einem der Ansprüche 5 bis 7, **dadurch gekennzeichnet, dass** der Schritt der Oxidation auf feuchtem Wege durchgeführt wird bei einer Temperatur zwischen 150°C und etwa 300°C und bei einem Druck zwischen 15 bar und 160 bar.

9. Behandlungslinie für Schlachthaus-Abwässer, enthaltend eine Klärstation (1), wobei die Abwässer zu einem Teil aus Schlämmen aus der Klärstation und zum anderen Teil aus wässrigen Abwässern bestehen, und wobei die Behandlungslinie eine Behandlungseinrichtung umfasst zum Behandeln der Schlämme zusammen mit Tiermehlen und/oder tierischen Fetten durch ein Verfahren nach den Ansprüchen 4 bis 8, wobei die Einrichtung Mittel zum thermischen Behandeln (2) der Mischung von Mehlen/Fetten besitzt, einschließlich zumindest eines Verdauungsapparats, der betrieben wird zur Bildung von Biogas, und von Leitmitteln dieses Biogases zu den wässrigen Abwässern, um sie zu säubern.

## Claims

1. Process for the treatment of flours and/or animal fats, comprising steps that consist in:
- mixing said flours and/or animal fats with residual sludges originating from sewage purification plants; and
- subjecting said mixture to a thermal sanitization treatment at a temperature above 135°C.

2. Process for the treatment of flours and/or animal fats according to Claim 1, **characterized in that** said mixture comprises between about 10% and 40% by dry weight of flours and/or animal fats.

3. Process for the treatment of flours and/or animal fats according to Claim 1 or 2, **characterized in that** said thermal treatment step comprises at least one step for thermal hydrolysis of said mixture by the injection of steam into it.

4. Process for the treatment of flours and/or animal fats according to Claim 3, **characterized in that** said thermal hydrolysis step is followed by at least one step for digestion of the hydrolysed sludges to produce a biogas and digested sludges.

5. Process for the treatment of flours and/or animal fats according to one of Claims 1 to 4, **characterized in that** said thermal treatment step comprises a wet oxidation step.

6. Process for the treatment of flours and/or animal fats according to Claims 4 and 5, **characterized in that** said wet oxidation step is carried out on said digested sludges.

7. Process for the treatment of flours and/or animal fats according to Claim 5, **characterized in that** said wet oxidation step is carried out on said mixture.

8. Process for the treatment of flours and/or animal fats according to any one of Claims 5 to 7, **characterized in that** said wet oxidation step is carried out at a temperature of between 150°C and about 300°C and at a pressure of between 15 bar and 160 bar.

9. System for treatment of the effluents originating from slaughterhouses integrating a purification plant (1), said effluents consisting on the one hand of sludges originating from said purification plant, and on the other hand of aqueous effluents, said system comprising an installation for the treatment of said sludges together with flours and/or animal fats by means of the process according to Claims 4 to 8, said installation comprising means (2) for thermal treatment of the sludge/flour mixture, including at least one digester (4), resulting in the formation of biogas, and means for passing said biogas into said aqueous effluents in order to sanitize them.
